# EUROPÄISCHE PATENTSCHRIFT

(11) **EP 3 307 159 B1**
(45) Veröffentlichungstag und Bekanntmachung des Hinweises auf die Patenterteilung: **08.04.2020**
(21) Anmeldenummer: 16727663.3
(22) Anmeldetag: 31.05.2016
(51) Int. Cl.: A61B 5/11, A61B 5/0488, A61F 2/68, G06F 3/03, A61F 2/54, G06F 3/01, A61B 5/00, A61F 2/70

(54) **VERFAHREN SOWIE VORRICHTUNG ZUR BESTIMMUNG EINER GEWOLLTEN BEWEGUNG EINER GLIEDMASSE**
METHOD AND DEVICE FOR DETERMINING A DESIRED MOVEMENT OF A LIMB
PROCÉDÉ ET DISPOSITIF DE DÉTERMINATION D'UN MOUVEMENT VOLONTAIRE D'UN MEMBRE

(30) Priorität: 10.06.2015 DE 102015210638
(43) Veröffentlichungstag der Anmeldung: 18.04.2018
(73) Patentinhaber: Deutsches Zentrum für Luft- und Raumfahrt e.V., 51147 Köln (DE)
(72) Erfinder: NISSLER, Christian, 82110 Germering (DE); CASTELLINI, Claudio, 82205 Gilching (DE)
(74) Vertreter: dompatent von Kreisler Selting Werner - Partnerschaft von Patent- und Rechtsanwälten mbB
(86) Internationale Anmeldenummer: PCT/EP2016/062269
(87) Internationale Veröffentlichungsnummer: WO 2016/198286

(56) Entgegenhaltungen:
- EP-A1- 2 506 807
- WO-A1-2014/005974
- WO-A1-2014/098494
- US-A1- 2012 004 579

## Beschreibung

Die Erfindung betrifft ein Verfahren sowie eine Vorrichtung zur Bestimmung einer gewollten Bewegung einer Gliedmaße.

Gliedmaßen des menschlichen Körpers werden durch Muskeln bewegt. Dabei sind die Muskeln der jeweiligen Gliedmaße stets proximal also zum Körperzentrum hin angeordnet, während die durch den Muskel bewegte Gliedmaße stets distal von den bewegenden Muskeln, also weiter entfernt vom Körper angeordnet ist. Durch die Erfassung der Muskelbewegung ist es möglich Rückschlüsse auf die gewollte Bewegung der entsprechenden Gliedmaße zu ziehen. Dies ist auch möglich, falls die gewollte Bewegung zwar muskulär initiiert wird, jedoch keine tatsächliche Bewegung der Gliedmaße erfolgt beispielsweise aufgrund einer amputierten Gliedmaße, einer Inhibition der Gliedmaße oder der Behinderung der Gliedmaße.

So können beispielsweise durch die Erfassung der Bewegung der Muskeln im Unterarm Rückschlüsse auf die gewollte Bewegung der Finger gezogen werden. Dies ist beispielsweise wünschenswert für die Ansteuerung von modernen Prothesen, also einer künstlichen Hand.

Grundsätzlich lassen sich drei Methoden zur Erkennung der Muskelbewegung unterscheiden. So können beispielsweise Kraft-Druck-Sensoren vorgesehen sein. Hier wird versucht, die Bewegung des Muskels anhand der Kraft, die dieser auf einen direkt an der Hautoberfläche angebrachten Sensor ausübt, zu messen. Beispiele hierfür sind sogenannte kraftfühlende Resistoren oder eine Anordnung von sehr vielen kraftmessenden Elementen (sogenannte tactels). Eine weitere Methode besteht in der Oberflächen-Elektromyographie (EMG). Hier werden durch die Messung der elektrischen Signale der Muskulatur Rückschlüsse auf die gewollte Bewegung der Gliedmaßen geschlossen. In einer dritten Methode wird Ultraschall als bildgebendes Verfahren verwendet, welches die Bewegung der Muskeln abbildet und Rückschlüsse auf die gewollte Bewegung der Gliedmaße erlaubt.

Nachteilig an diesen Methoden ist jedoch, dass alle eine direkte Anbringung der Sensoren auf die Haut voraussetzen. Damit verbunden ergeben sich Nachteile, da diese Sensoren exakt positioniert werden müssen und beispielsweise ein Verrutschen der Sensoren zu einer Abweichung des Signals führt. Durch den Kontakt kann es darüber hinaus zu Beschädigungen der Haut an den Kontaktflächen führen. Somit muss einerseits geschultes Personal vorhanden sein zur Anbringung der Sensoren, andererseits besteht eine hohe Fehlerquelle in der korrekten Positionierung bzw. dem Verrutschen der Sensoren und wiederum ist die Dauer der Erfassung durch den unmittelbaren Hautkontakt der Sensoren beschränkt.

Des Weiteren gibt es Ermüdungserscheinungen der Signalqualität über längere Zeiten (z.B. bei EMG Signalen) und Probleme der Ultraschallkopplung an der Hautoberfläche. Außerdem sind die verwendeten Sensoren sehr komplex und dadurch teuer.

EP 2 506 807 A1 beschreibt eine Vorrichtung zum Steuern von Prothese, wobei mittels nichtinvasiver Sensoren Muskelkontraktionen detektiert werden, auf Basis derer dann die Prothese gesteuert wird.

WO 2014/005974 A1 beschreibt ein Biofeedbacksystem, bei dem durch Sensoren die Aktivität von Muskeln erfasst wird und dem Benutzer ein Feedback gegeben wird.

Eine weitere Vorrichtung zum Steuern einer Prothese ist bekannt aus WO 201/098494 A1. Hierbei werden Augenbewegungen des Nutzers zur Bewegung der Prothese verwendet.

US 2012/004579 A1 beschreibt ein Verfahren zum Trainieren von handamputierten Personen. Beabsichtigte Bewegungen einer amputierten Gliedmaße können durch ein Kamerasystem erfasst werden, indem Marker an der Schulter des Patienten angebracht werden. Auf Basis der erfassten Eingabebefehle wird eine virtuell komplettierte Gliedmaße auf einer Anzeigevorrichtung dargestellt.

Aufgabe der Erfindung ist es, ein Verfahren sowie eine Vorrichtung zur Erfassung einer gewollten Bewegung einer Gliedmaße zu schaffen, welche berührungsfrei arbeitet.

Die Aufgabe wird gelöst durch das Verfahren gemäß Anspruch 1 sowie die Vorrichtung gemäß Anspruch 8.

In dem erfindungsgemäßen Verfahren zur Bestimmung einer gewollten Bewegung einer Gliedmaße wird ein Ausschnitt einer Körperoberfläche beobachtet. Bei der gewollten Bewegung ist es nicht erforderlich, dass eine tatsächliche Bewegung der Gliedmaße erfolgt. So kann die tatsächliche Gliedmaße beispielsweise durch Fehlen der Gliedmaße, Inhibition oder Behinderung der Gliedmaße verhindert oder abgeschwächt sein. Bei der Gliedmaße handelt es sich dabei insbesondere um eine Gliedmaße des menschlichen Körpers. Der Ausschnitt der Körperoberfläche ist dabei so gewählt, dass an der Stelle des Ausschnitts zumindest ein Teil der die Gliedmaße bewegenden Muskulatur vorhanden ist.

Bei der Beobachtung der Körperoberfläche findet in dem erfindungsgemäßen Verfahren keine Berührung der Körperoberfläche statt. In dem Ausschnitt werden erfindungsgemäß die erfassbaren Muskelbewegungen optisch erfasst. Nachfolgend werden die so erfassten Muskelbewegungen ausgewertet und hieraus die gewollte Bewegung der betreffenden Gliedmaße ermittelt. Hierbei findet keine direkte Beobachtung der entsprechenden Gliedmaße statt. Vielmehr soll eine Vorhersage aufgrund der beobachteten Muskelbewegung getroffen werden, welche Bewegung die Person vornehmen möchte.

Durch die optische Erfassung der Muskelbewegung ist das Verfahren berührungslos. Eine Verbindung eines Sensors mit dem Ausschnitt der beobachteten Körperoberfläche ist nicht erforderlich. Insbesondere wird zur optischen Erfassung eine Kamera verwendet. Hierbei sind insbesondere keine besonderen Anforderungen an die Kamera zu stellen, so dass eine Kamera mit einer Auflösung von 1280 x 720 Pixeln bereits als ausreichend angesehen werden kann. Das Erfassen der Bilder erfolgt hierbei vorzugsweise mit einer Frequenz von 15 Bildern pro Sekunde (frames per second) oder mehr. Somit ergibt sich ein einfacher Aufbau mit preisgünstigen Komponenten. Auch Probleme wie Ermüdungserscheinungen mit EMG Sensoren und der schwierigen dauerhaften Ankopplung von Ultraschallsensoren an der Hautoberfläche sind im erfindungsgemäßen Verfahren nicht vorhanden. Auch kann eine gesundheitliche Gefährdung ausgeschlossen werden, da keine direkte Anbringung von Sensorik und keine Einbringung von Energie in den Körper stattfindet.

Insbesondere kann durch das Verfahren gleichzeitig eine Vielzahl von Muskelbewegungen erfasst werden. Da ein gesamter Ausschnitts einer Köperoberfläche beobachtet wird, erfolgt keine punktuelle Detektion einer Muskelbewegung wie es bei den üblichen und bekannten Methoden der Fall ist. Die gleichzeitige Erfassung einer Vielzahl von Muskelbewegungen erlaubt einerseits eine präzisere Aussage über die gewollte Bewegung, andererseits können hierdurch komplexe Bewegungen, welche mehr als einen Muskel erfordern, ermittelt werden.

Vorzugsweise wird durch das Verfahren gleichzeitig eine Vielzahl von gewollten Bewegungen mehrerer Gliedmaßen ermittelt. Insbesondere falls der Unterarm beobachtet wird, kann es sich bei der Vielzahl von gewollten Bewegungen um die gewollten Bewegungen der Finger handeln, welche durch das Verfahren ermittelt werden. Somit ist es nicht erforderlich sich im Beispiel des beobachteten Unterarms lediglich auf die Bewegung eines Fingers zu beschränken. Vielmehr können die Bewegungen aller Finger gleichzeitig beobachtet werden.

Vorzugsweise sind auf dem beobachteten Ausschnitt der Körperoberfläche Markierungen angebracht zur Erfassung der Muskelbewegungen. Die Markierungen Vereinfachen lediglich die Erfassung der Muskelbewegung und die Auswertung. Dabei können die Markierungen auf den Ausschnitt der Köperoberfläche aufgeklebt sein, aufgemalt werden oder insbesondere durch Tätowierungen unmittelbar in die Körperoberfläche eingebracht werden.

Vorzugsweise sind auf dem beobachteten Ausschnitt der Körperoberfläche Emitter und/oder Reflektoren angebracht zur Erfassung der Muskelbewegungen. Hierdurch wird die Erfassung der Muskelbewegungen und die Ermittlung der gewollten Bewegung vereinfacht. Insbesondere handelt es sich bei dem Emitter um IR-Emitter, so dass diese von einer konventionellen Kamera erfasst werden können.

Vorzugsweise werden bei der Auswertung der erfassten Muskelbewegung ein Tiefpassfilter, ein Hochpassfilter oder ein Bandpassfilter verwendet. Hierdurch werden beispielsweise DC-Komponenten und/oder hochfrequente Komponenten aus dem Signal entfernt. Insbesondere wird hierzu ein Butterworth Bandpassfilter zweiter Ordnung verwendet.

Insbesondere wird bei der Auswertung eine Bilderfassung durchgeführt, so dass die Bewegung der Körperoberfläche beziehungsweise der darauf angebrachten Markierungen bevorzugt in allen drei Raumrichtungen sowie in den drei Rotationsrichtungen - also 6D - erfasst werden. Möglich ist hierbei jedoch auch nur die Erfassung von beispielsweise zwei Translationsrichtungen- also 2D - oder verschiedene Kombinationen aus Translation und Rotation. Aus der erfassten Bewegung der Muskeln beziehungsweise der auf der Körperoberfläche angebrachten Markierungen lässt sich die gewollte Bewegung der betreffenden Gliedmaßen ermitteln.

Vorzugsweise wird eine Verknüpfung zwischen Muskelbewegung und gewollter Bewegung erzeugt bzw. abgeleitet durch Beobachtung einer Vielzahl von Muskelbewegungen, die durch eine vorgegebene Bewegung erzeugt werden. Hierdurch kann eine Muskelbewegung einer vorgegebenen und damit bekannten gewollten Bewegung zugeordnet werden. Somit wird durch eine Auswertvorrichtung die Verknüpfung zwischen der beobachteten Muskelbewegung und der bekannten gewollten Bewegung erzeugt. Diese Korrelation zwischen der erfassten Muskelbewegung und der bekannten gewollten Bewegung wird in der folgenden Auswertung bei unbekannter gewollter Bewegung zur Bestimmung derselben herangezogen. Somit findet ein Anlernen der Auswertvorrichtung statt, welche einmal durchgeführt werden muss, um im Weiteren unbekannte gewollte Bewegungen der betreffenden Gliedmaßen zu bestimmen.

Vorzugsweise wird die Verknüpfung zwischen Musekelbewegung und gewollter Bewegung durch eine Ridge Regression (A.E. Hoerl and R.W. Kennard, "Ridge regression: Biased estimation for nonorthogonal problems", Technometrics, Bd. 12, S. 55-67,1970) durchgeführt, welche ein lineares Modell an die erfassten Daten anpasst. Aus dieser Verknüpfung lassen sich insbesondere unbekannte gewollte Bewegungen aus einer beobachteten Muskelbewegung ableiten.

Die vorliegende Erfindung betrifft darüber hinaus eine Vorrichtung zur Ermittlung einer gewollten Bewegung einer Gliedmaße mit einer Kamera, die auf einen Ausschnitt einer Körperoberfläche gerichtet ist. Durch die Kamera wird eine Muskelbewegung in dem Ausschnitt erfasst. Weiter ist erfindungsgemäß mit der Kamera eine Auswertvorrichtung verbunden. Durch die Auswertvorrichtung wird die optisch erfasste Muskelbewegung ausgewertet. Dabei umfasst die Auswertung eine Bestimmung der gewollten Bewegung der Gliedmaße. Mit der Auswertevorrichtung wiederum ist eine Ausgabevorrichtung verbunden zu Ausgabe des Auswertungsergebnisses und insbesondere zur Ausgabe der gewollten Bewegung.

Vorzugsweise ist die Vorrichtung weitergebildet entsprechend dem vorstehend beschriebenen Verfahren.

Vorzugsweise weist das Verfahren Merkmale der Vorrichtung auf.

Die vorliegende Erfindung betrifft weiter die Verwendung der vorstehend beschriebenen Vorrichtung zur Steuerung einer Prothese der entsprechenden Gliedmaße. Hier ist die Ausgabevorrichtung der erfindungsgemäßen Vorrichtung mit der Prothese, bei der es sich beispielsweise um eine Handprothese handelt, verbunden. Durch die Erfassung der gewollten Bewegung kann diese gewollte Bewegung in eine tatsächliche Bewegung der Prothese umgesetzt werden.

Weiter betrifft die Erfindung die Verwendung der vorstehend beschriebenen Vorrichtung zur Steuerung einer Maschine, insbesondere eines Roboter. Somit können die gewollten Bewegungen insbesondere der Hand ermittelt werden und im Rahmen einer Mensch-Maschine Interaktion benutzt werden, um die Maschine und vorzugsweise den Roboter geeignet anzusteuern. Beispielsweise handelt es sich bei dem Roboter um einen Assistenzroboter, der einem Handamputierten beispielsweise ein Glas greift und reicht. Jedoch ist auch bei gesunden Personen, die Verwendung der Vorrichtung möglich im Sinne eines Eingabegerätes zur Steuerung von Maschinen.

Weiter betrifft die Erfindung die Verwendung der vorstehen Vorrichtung zur Steuerung und Darstellung einer Gliedmaße in einer virtuellen Realität. So können beispielsweise in der virtuellen Realität Hände dargestellt werden, welche durch die Erfassung der Muskelbewegungen gesteuert und bewegt werden. Eine tatsächliche Bewegung beispielsweise der Hand ist nicht erforderlich.

Nachfolgend wird die Erfindung anhand einer bevorzugten Ausführungsform in Bezug auf die angefügten Abbildungen beschrieben.

Es zeigen:
- Fig. 1: eine erfindungsgemäße Vorrichtung zur Bestimmung einer gewollten Bewegung am Beispiel der gewollten Bewegung einer Hand,
- Fig. 2: ein schematischer Ablauf des erfindungsgemäßen Verfahrens und
- Fig. 3: ein schematischer Ablauf zur Bestimmung einer Verknüpfung zwischen gewollter Bewegung und erfasster Muskelbewegung.

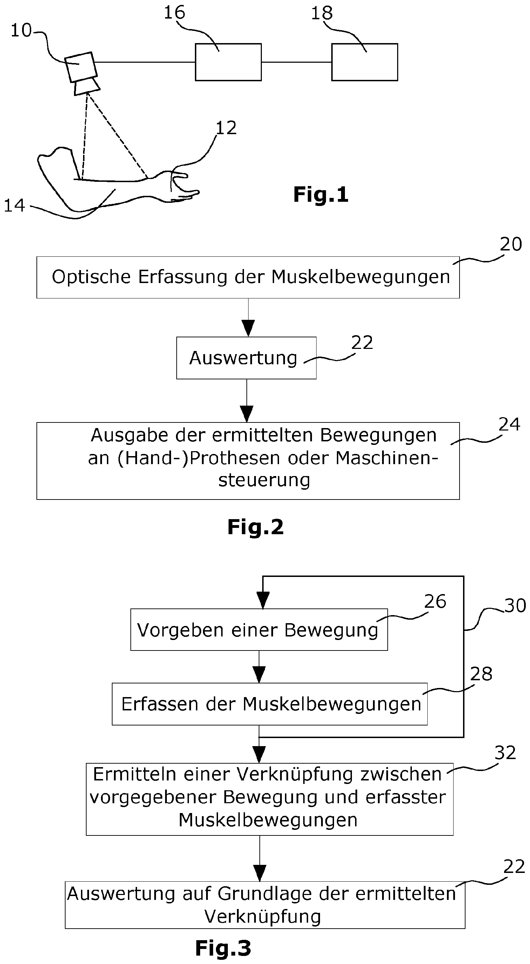

Erfindungsgemäß weist die Vorrichtung zur Ermittlung einer gewollten Bewegung einer Gliedmaße eine Kamera 10 auf. Im in Fig. 1 dargestellten Beispiel handelt es sich bei der Gliedmaße, welche bewegt werden soll, um eine menschliche Hand 12. Durch die Kamera 10 wird dabei ein Ausschnitt der Körperoberfläche beobachtet, an welchem zumindest ein Teil der die Gliedmaße bewegenden Muskulatur vorhanden ist. Im Beispiel der Hand 12 wird hierbei durch die Kamera 10 der Unterarm 14 beobachtet, um Muskelbewegungen optisch zu erfassen. Hierbei findet keine Berührung des Unterarms durch einen Sensor oder dergleichen statt.

Die Kamera 10 ist mit einer Auswertvorrichtung 16 verbunden. Durch die Auswertvorrichtung 16 werden die durch die Kamera 10 erfassten Bilder ausgewertet und hieraus die Muskelbewegung am Unterarm 14 bestimmt. Aus der so bestimmten Muskelbewegung der Muskeln am Unterarm 14 wird durch die Auswertvorrichtung 16 die gewollte Bewegung der Hand 12 und/oder der Finger ermittelt. Dies ist möglich, da eine eindeutige Verbindung zwischen den Muskeln und Hand bzw. den einzelnen Fingern besteht, so das deterministisch durch die Bewegung eines bestimmten Muskels vorhergesagt werden kann, welche Finger oder welcher Teil der Hand bewegt werden sollt. Dies trifft auf alle Muskeln zu, welche stets exakt eine Bewegungsrichtung aufweisen. Selbstverständlich ist eine Interaktion der einzelnen Muskeln zur Erzeugung einer komplexen Bewegung einer Gliedmaße ebenfalls mit umfasst.

Mit der Auswertvorrichtung 16 ist eine Ausgabevorrichtung 18 verbunden, durch die Ausgabevorrichtung 18 wird die gewollte Bewegung, im vorliegenden Beispiel der Hand 12, ausgegeben. Die Ausgabevorrichtung 18 kann ihrerseits wiederum beispielsweise verbunden sein mit einer Prothese, welche sodann die gewollte Bewegung ausführt oder aber in einer anderen Anwendung kann die Auswertvorrichtung 18 mit einer anderen Maschine verbunden sein, welche sodann eine entsprechende Bewegung ausführt.

Auch wenn in Fig. 1 ein Beispiel der Vorrichtung anhand der Hand dargestellt ist, ist dieses selbstverständlich auf jede andere Gliedmaße übertragbar.

Im erfindungsgemäßen Verfahren, schematisch dargestellt in der Fig. 2 wird zunächst eine Muskelbewegung optisch erfasst 20. Die Muskelbewegung wird dabei erfasst an der Köperoberfläche innerhalb eines beobachteten Ausschnitts. Der Ausschnitt ist dabei an einer Stelle angeordnet, an welcher sich zumindest ein Teil der die Gliedmaßen bewegenden Muskulatur vorhanden ist. Die so erfasste Muskelbewegung wird ausgewertet 22, dies umfasst die Ermittlung einer gewollten Bewegung der betreffenden Gliedmaße. In einem weiteren Schritt 24 kann die so ermittelte Bewegung ausgegeben werden beispielsweise an eine Prothese, welche die zu bewegende Gliedmaße ersetzt oder an eine Maschinensteuerung.

Es ist möglich zur Auswertung 22 die Auswertvorrichtung 16 geeignet anzulernen. Hierdurch werden der Person Bewegungen der Gliedmaße zunächst vorgegeben 26. Die Person führt die vorgegebenen Bewegungen aus. Ob es sich dabei um eine tatsächliche Bewegung der Gliedmaße handelt oder, falls beispielsweise diese Gliedmaße fehlt, um lediglich die Aktivierung der entsprechenden Muskeln ist dabei unerheblich. Es werden weiter die Muskelbewegungen erfasst, welche aufgrund der vorgegebenen Bewegung erzeugt werden 28. Die vorgenannten Schritte werden ausreichend oft wiederholt 30. Sobald eine genügend große Anzahl an Wiederholungen vorliegt wird hieraus eine Verknüpfung zwischen vorgegebener Bewegung und erfasster Muskelbewegung ermittelt 32. Hierbei kann es sich beispielsweise um eine Regressionsanalyse der Bewegung handeln. Im Nachfolgenden findet die Auswertung 22 auf Grundlage der so ermittelten Verknüpfung statt. Durch dieses Verfahren kann auf relativ einfache Weise eine personenbezogene Auswertung durch die individuelle Verknüpfung von Bewegung der Gliedmaße und Muskelbewegung erzeugt werden.

## Patentansprüche

1. Verfahren zur Bestimmung einer gewollten Bewegung einer nicht mehr vorhandenen Gliedmaße (12), bei welchem
ein Ausschnitt einer Körperoberfläche, an der Stelle, an welcher zumindest ein Teil der die Gliedmaße bewegenden Muskulatur vorhanden ist, beobachtet wird,
die im Ausschnitt stattfindenden Muskelbewegungen optisch erfasst werden (20),
die so erfassten Muskelbewegungen ausgewertet werden (22) und
hieraus die gewollte Bewegung der betreffenden Gliedmaße (12) ermittelt wird,
**dadurch gekennzeichnet, dass**
eine Verknüpfung zwischen einer gewollten Bewegung der nicht mehr vorhandenen Gliedmaße und der im Ausschnitt erfassten Muskelbewegung wie folgt bestimmt wird:
Vorgeben einer Bewegung der Gliedmaße (26) und Ausführen der vorgegebenen Bewegung durch die Person, wobei hierbei aufgrund der fehlenden Gliedmaße lediglich die entsprechenden Muskeln aktiviert werden,
Erfassen der Muskelbewegungen, welche aufgrund der vorgegebenen Bewegung erzeugt werden (28),
Wiederholen (30) der vorangegangenen Verfahrensschritte (26) und (28),
Ermitteln einer Verknüpfung zwischen vorgegebener Bewegung und den erfassten Muskelbewegungen (32),
wobei die Auswertung (22) der erfassten Muskelbewegungen auf Grundlage dieser ermittelten Verknüpfung erfolgt,
wobei die erfassten Muskelbewegungen zur Steuerung einer Prothese einer Gliedmaße oder einer Maschine und insbesondere eines Roboters verwendet werden.

2. Verfahren nach Anspruch 1, bei welchem gleichzeitig eine Vielzahl von Muskelbewegungen erfasst werden.

3. Verfahren nach Anspruch 1 oder 2, bei welchem gleichzeitig eine Vielzahl von gewollten Bewegungen mehrerer Gliedmaßen (12) ermittelt werden.

4. Verfahren nach einem der Ansprüche 1 bis 3, bei welchem der Unterarm (14) beobachtet wird und gewollte Bewegungen der Hand (12) und/oder der Finger ermittelt werden.

5. Verfahren nach einem der Ansprüche 1 bis 4, bei welchem in der Auswertung der erfassten Muskelbewegungen ein Tiefpassfilter, ein Hochpassfilter oder ein Bandpassfilter verwendet wird.

6. Verfahren nach einem der Ansprüche 1 bis 5, bei welchem auf dem beobachteten Ausschnitt der Körperoberfläche IR-Emitter angebracht werden zur Erfassung der Muskelbewegungen.

7. Verfahren nach einem der Ansprüche 1 bis 6, bei der eine Auswertvorrichtung durch Beobachtung der Muskelbewegung bei einer Vielzahl vorgegebenen Bewegungen (26) eine Verknüpfung zwischen Muskelbewegung und bekannter gewollter Bewegung erzeugt wird (28) und im Folgenden bei der Auswertung (22) der erfassten Muskelbewegung diese Verknüpfung herangezogen wird zur Ermittlung einer unbekannten gewollten Bewegung.

8. Vorrichtung zur Ermittlung einer gewollten Bewegung einer nicht mehr vorhandenen Gliedmaße, mit einer Auswertvorrichtung (16) und
einer mit der Auswertvorrichtung (16) verbundenen Ausgabevorrichtung (18) zur Ausgabe eines Auswertungsergebnisses,
**gekennzeichnet durch**
eine auf einen Ausschnitt einer Körperoberfläche gerichtete Kamera (10) zur Erfassung einer Muskelbewegung,
wobei die Auswertvorrichtung (16) mit der Kamera (10) verbunden ist zur Auswertung der optisch erfassten Muskelbewegung, welche eine Bestimmung der gewollten Bewegung umfasst,
**dadurch gekennzeichnet, dass**
eine Verknüpfung zwischen einer gewollten Bewegung der nicht mehr vorhandenen Gliedmaße und der im Ausschnitt erfassten Muskelbewegung wie folgt bestimmt wird:
Vorgeben einer Bewegung der Gliedmaße (26) und Ausführen der vorgegebenen Bewegung durch die Person, wobei hierbei aufgrund der fehlenden Gliedmaße lediglich die entsprechenden Muskeln aktiviert werden,
Erfassen der Muskelbewegungen, welche aufgrund der vorgegebenen Bewegung erzeugt werden (28),
Wiederholen (30) der vorangegangenen Verfahrensschritte (26) und (28),
Ermitteln einer Verknüpfung zwischen vorgegebener Bewegung und den erfassten Muskelbewegungen (32),
wobei die Auswertung (22) der erfassten Muskelbewegungen auf Grundlage dieser ermittelten Verknüpfung erfolgt,
wobei die erfassten Muskelbewegungen zur Steuerung einer Prothese einer Gliedmaße oder einer Maschine und insbesondere eines Roboters verwendet werden.

9. Vorrichtung nach Anspruch 8 zur Durchführung des Verfahrens nach einem der Ansprüche 1 bis 7.

## Claims

1. A method for determining a desired movement of a limb no longer existing (12), where
a section of a body surface area at the place where at least a portion of the musculature moving the limb is present is observed,
the muscular movements taking place in said section are optically captured (20),
the thus captured muscular movements are evaluated (22), and
thereof the desired movement of the limb (12) concerned is identified, **characterized in that**
a linkage between a desired movement of the limb no longer existing and the muscular movement captured in the section is determined as follows:
predetermining a movement of the limb (26) and performing the predetermined movement by the person, wherein here due to the missing limb merely the corresponding muscles are activated,
capturing the muscular movements produced by the predetermined movement (28),
repeating (30) the preceding method steps (26) and (28),
identifying a linkage between the predetermined movement and the captured muscular movements (32),
wherein the evaluation (22) of the captured muscular movements is performed on the basis of this identified linkage,
wherein the captured muscular movements are used for controlling a prothesis of a limb or a machine and in particular a robot.

2. The method according to claim 1, wherein a plurality of muscular movements are simultaneously captured.

3. The method according to claim 1 or 2, wherein a plurality of desired movements of several limbs (12) are simultaneously identified.

4. The method according to any one of claims 1 to 3, wherein the forearm (14) is observed and desired movements of the hand (12) and/or the fingers are identified.

5. The method according to any one of claims 1 to 4, wherein for evaluation of the captured muscular movements a low-pass filter, a high-pass filter or a band-pass filter is used.

6. The method according to any one of claims 1 to 5, wherein IR emitters are attached to the observed section of the body surface area for capturing the muscular movements.

7. The method according to any one of claims 1 to 6, wherein an evaluation device generates a linkage between a muscular movement and a known desired movement (28) by observing the muscular movement during a plurality of predetermined movements (26) and subsequently, during an evaluation (22) of the captured muscular movement, the linkage is used for identifying an unknown desired movement.

8. A device for determining a desired movement of a limb no longer existing, comprising
an evaluation device (16), and
an output device (18) connected to the evaluation device (16) for outputting an evaluation result,
**characterized by**
a camera (10) directed to a section of the body surface area for capturing a muscular movement,
wherein the evaluation device (16) is connected to the camera (10) for evaluation of the optically captured muscular movement comprising a determination of the desired movement,
**characterized in that**
a linkage between a desired movement of the limb no longer existing and the muscular movement captured in the section is determined as follows:
predetermining a movement of the limb (26) and performing the predetermined movement by the person, wherein here due to the missing limb merely the corresponding muscles are activated,
capturing the muscular movements produced by the predetermined movement (28),
repeating (30) the preceding method steps (26) and (28),
identifying a linkage between the predetermined movement and the captured muscular movements (32),
wherein the evaluation (22) of the captured muscular movements is performed on the basis of this identified linkage,
wherein the captured muscular movements are used for controlling a prothesis of a limb or a machine and in particular a robot.

9. The device according to claim 8 for carrying out the method according to any one of claims 1 to 7.

## Revendications

1. Procédé de détermination d'un mouvement volontaire d'un membre (12) qui n'est plus présent, selon lequel
une portion d'une surface de corps est observée à l'endroit où se trouve au moins une partie de la musculature mouvant le membre,
les mouvements musculaires prenant place dans la portion sont détectés optiquement (20),
les mouvements musculaires ainsi détectés sont interprétés (22) et
le mouvement volontaire du membre concerné est déterminé à partir de ceux-ci (12),
**caractérisé en ce qu'**une association entre un mouvement volontaire du membre absent et le mouvement musculaire détecté dans la portion est déterminée comme suit :
définition d'un mouvement du membre (26) et exécution du mouvement défini par la personne, dans lequel à cet effet seuls les muscles correspondants sont activés en raison du membre manquant,
détection des mouvements musculaires produits en raison du mouvement défini (28),
répétition (30) des étapes de procédé précédentes (26) et (28),
détermination d'une association entre le mouvement défini et les mouvements musculaires détectés (32),
dans lequel l'interprétation (22) des mouvements musculaires détectés s'effectue sur la base de cette association déterminée,
dans lequel les mouvements musculaires détectés sont utilisés pour commander une prothèse d'un membre ou une machine et en particulier un robot.

2. Procédé selon la revendication 1, lors duquel une pluralité de mouvements musculaires sont détectés simultanément.

3. Procédé selon la revendication 1 ou 2, lors duquel une pluralité de mouvements volontaires de plusieurs membres (12) sont déterminés simultanément.

4. Procédé selon l'une des revendications 1 à 3, lors duquel l'avant-bras (14) est observé et les mouvements volontaires de la main (12) et/ou des doigts sont déterminés.

5. Procédé selon l'une des revendications 1 à 4, lors duquel un filtre passe-bas, un filtre passe-haut ou un filtre passe-bande est utilisé dans l'interprétation des mouvements musculaires détectés.

6. Procédé selon l'une des revendications 1 à 5, lors duquel un émetteur IR est arrangé sur la portion de la surface de corps observée afin de détecter les mouvements musculaires.

7. Procédé selon l'une des revendications 1 à 6, lors duquel un dispositif d'interprétation par observation du mouvement musculaire lors d'une pluralité de mouvements définis (26) obtient une association (28) entre mouvement musculaire et mouvement volontaire connu et par la suite lors de l'interprétation (22) du mouvement musculaire détecté cette association est utilisée pour déterminer un mouvement volontaire inconnu.

8. Dispositif de détermination d'un mouvement volontaire d'un membre qui n'est plus présent, doté
d'un dispositif d'interprétation (16) et
d'un dispositif d'émission (18) relié au dispositif d'interprétation (16) permettant d'émettre un résultat d'interprétation,
**caractérisé par**
une caméra (10) dirigée sur une portion d'une surface de corps permettant de saisir un mouvement musculaire,
dans lequel le dispositif d'interprétation (16) est relié à la caméra (10) afin d'interpréter le mouvement musculaire détecté optiquement, ce qui comprend une détermination du mouvement volontaire,
**caractérisé en ce que**
une association entre un mouvement volontaire du membre absent et le mouvement musculaire détecté dans la portion est déterminée comme suit :
définition d'un mouvement du membre (26) et exécution du mouvement défini par la personne, dans lequel à cet effet seuls les muscles correspondants sont activés en raison du membre manquant,
détection des mouvements musculaires produits en raison du mouvement défini (28),
répétition (30) des étapes de procédé précédentes (26) et (28),
détermination d'une association entre le mouvement défini et les mouvements musculaires détectés (32),
dans lequel l'interprétation (22) des mouvements musculaires détectés s'effectue sur la base de cette association déterminée,
dans lequel les mouvements musculaires détectés sont utilisés pour commander une prothèse d'un membre ou une machine et en particulier un robot.

9. Dispositif selon la revendication 8 destiné à effectuer un procédé selon l'une des revendications 1 à 7.
